(19) Europäisches Patentamt
European Patent Office
Office européen des brevets

(11) EP 3 842 028 A1

(12) # EUROPEAN PATENT APPLICATION

(43) Date of publication:
**30.06.2021 Bulletin 2021/26**

(21) Application number: **19219354.8**

(22) Date of filing: **23.12.2019**

(51) Int Cl.:
***A61K 8/81*** *(2006.01)* ***A61Q 19/00*** *(2006.01)*
***A61K 31/785*** *(2006.01)* ***A61P 17/00*** *(2006.01)*
***A61P 17/18*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Unilever Global IP Ltd**
**Wirral, CH62 4ZD (GB)**

(72) Inventors:
- **BHOGAL, Ranjit Kaur**
  **Bedforshire, MK44 1 LQ (GB)**
- **MESSENGER, David James**
  **Bedforshire, MK44 1 LQ (GB)**

(74) Representative: **James, Helen Sarah**
**Unilever PLC**
**Unilever Patent Group**
**Bronland 14**
**6708 WH Wageningen (NL)**

(54) **COMPOSITION FOR REDUCING SKIN DAMAGE FROM POLLUTION**

(57) Disclosed is a polymer comprising an acrylamidopropyltrimonium salt for use in the prevention of pollutant-mediated damage to human skin.

EP 3 842 028 A1

## Description

### Background of the invention

The present invention relates to the use of certain compounds to prevent pollution damage to the skin of an individual. More specifically it relates to the use of compounds and compositions for the prevention of lipoperoxidation-based damage to the skin.

### BACKGROUND OF THE INVENTION

Air pollution is known to have major effects on the human skin. Pollutants such as polycyclic aromatic hydrocarbons, volatile organic compounds, oxides, particulate matter, ozone and cigarette smoke affect the skin as it is the outermost barrier, causing damage by inducing oxidative stress.

Human skin acts as a biological shield against pro-oxidative chemicals and physical air pollutants and as such, exposure to these pollutants can have serious effects. For example, smoke contributes to premature aging and an increase in the incidence of psoriasis, acne and skin cancers and is also implicated in allergic skin conditions such as atopic dermatitis and eczema. Polyaromatic hydrocarbons are associated with extrinsic skin aging, pigmentation, cancers and acneiform eruptions. Volatile organic compounds have been associated with atopic dermatitis.

Many strategies are therefore recommended to address the damage caused to skin by pollution. A recent review by Mistry (Cosmetics 2017, 4, 57) lists the following possible approaches to address the effects of pollution on the skin: reducing particle load by cleansing or exfoliation; preventing deposition and penetration of pollutants on skin; restoring and strengthening the skin's protective barrier structure and function; reducing trans epidermal water loss to improve skin hydration; replenishing antioxidant reserves; reducing inflammation; controlling melanogenesis; promoting collagen/elastin synthesis; and protecting skin from UV.

Lipid peroxidation (lipoperoxidation) is known to be caused by pollution and is a crucial step in the pathogenesis of several disease states in adults and infants. Lipid peroxidation is a process generated mainly by the effect of several reactive oxygen-containing species (hydroxyl radical, hydrogen peroxide etc.). It can also be generated by the action of several phagocytes. The reactive oxygen species ("ROS") readily attack the polyunsaturated fatty acids of the fatty acid cell membrane, initiating a self-propagating chain reaction. Malondialdehyde (MDA) is one of several low molecular weight end products formed via the decomposition of certain primary and secondary lipid peroxidation products (Janero, Free Radic. Biol. Med. 1990; 9(6)515-540). MDA can thus be used as a marker of lipoperooxidation and can be conveniently assayed using thiobarbituric acid, with which it forms a fluorescent adduct. The destruction of membrane lipids and the end-products of such lipid peroxidation reactions are especially dangerous for the viability of cells, even tissues. Enzymatic (catalase, superoxide dismutase) and nonenzymatic (vitamins A and E) natural antioxidant defence mechanisms exist; however, these mechanisms may be overcome, allowing lipid peroxidation to take place. Since lipid peroxidation is a self-propagating chain-reaction, the initial oxidation of only a few lipid molecules can result in significant tissue damage.

Lipoperoxidation can occur in the skin. The skin is a complex tissue comprising many cell types, not only the keratinocytes that make up the surface. For example, the skin also includes sweat glands, sebaceous glands and hair follicles, and the cells making up these structures are also susceptible to lipoperoxidation. Thus, for instance, cells making up hair fibres (produced from the hair follicles) may also be damaged by pollution-mediated effects, such as lipoperoxidation.

It can therefore be appreciated that pollution can be addressed in many different ways and the present invention is directed to the prevention of lipoperoxidation-based damage to the skin through the use of barriers, specifically film-forming compositions.

In a study by Portugal-Cohen et al. (Clinical, Cosmetic and Investigational Dermatology 2017:10 185-193), two representative pollution models were studied using reconstructed epidermis: 1) mixture of pollutants containing heavy metals and atmospheric particulate matter and 2) ozone exposure. Dead Sea mineral-rich water and an anionic polysaccharide film former (PolluStop) were topically applied alone or in combination, and their protection against pollution was assessed by testing the levels of the inflammation markers interleukin 1a (IL-1a) and prostaglandin E2 (PGE2). It was found that exposure to the mixture of pollutants induced IL-1a release, which was attenuated by prior application of Dead Sea mineral-rich water and anionic polysaccharide alone or in combination. Ozone exposure induced IL-1a and PGE2 release. Prior application of Dead Sea mineral-rich water or anionic polysaccharide film former alone did not inhibit IL-1a and PGE2 overproduction caused by ozone exposure. However, when they were mixed together, inhibition of these inflammatory markers was observed.

US2017100311 generally relates to a skin treatment composition comprising an emulsion (e.g., a water-in-oil emulsion) comprising an oil phase comprising (a) an acrylate polymer and (b) a silicone resin, wherein (a) and (b) are dissolved in a non-volatile emollient oil, and wherein the composition is essentially free of any additional emulsifiers (i.e.,

beyond any already listed above). The composition can be configured to dry to a coating comprising at least 60 wt percent of the emollient oil. However, this disclosure does not deal with the prevention of lipoperoxidation-based damage to the skin through the use of a specific film-forming composition.

## Summary of the invention

The present invention is based on the discovery by the inventors that certain compounds, which are safe for topical application to humans, are effective in preventing or reducing damage to human tissue (especially human skin) mediated by pollutants.

Accordingly, in a first aspect, the invention provides a polymer comprising an acrylamidopropyltrimonium salt for use in the prevention of pollutant-mediated damage to human skin.

In a second aspect the invention provides a method for preventing pollutant-mediated damage to human skin, the method comprising the step of applying to the skin of a human subject an effective amount of a polymer comprising an acrylamidopropyltrimonium salt. In particular, the method is a method for cosmetic treatment of the skin by preventing pollution-mediated damage thereto.

The polymer preferably comprises polyacrylamidopropyltrimonium salt and in some embodiments the polymer consists entirely of polyacrylamidopropyltrimonium salt. In some embodiments the polymer may comprise a percentage of one or more other monomers.

In those embodiments in which the polymer may compromise other monomers in addition to acrylamidopropyltrimonium salt, it is preferred that only one additional monomer is incorporated into the polymer. An especially preferred additional monomer is acrylamide.

The polymer of use in the invention will typically comprise at least 50 mol% of acrylamidopropyltrimonium salt, and preferably will comprise from 51 to 100 mol% acrylamidopropyltrimonium salt.

Typically the polymer comprising the acrylamidopropyltrimonium salt should be present at a concentration of at least 1mg/ml in a composition to be applied to the skin, preferably at a concentration of at least 5 mg/ml, and more preferably at a concentration of at least 20mg/ml. A maximum concentration of the polymer comprising the acrylamidopropyltrimonium salt will typically be about 100mg/ml, so suitable concentration ranges encompass 1-100mg/ml, preferably 5-100mg/ml, more preferably 20-50mg/ml.

Preferably the salt is a halide, especially a chloride.

In particular, the polymer comprising the acrylamidopropyltrimonium salt inhibits or reduces lipid peroxidation mediated by pollutants.

The polymer comprising the acrylamidopropyltrimonium salt is preferably applied to the skin on the face and/or hands, but could also be applied to the skin on other parts of the body, especially where the skin might be exposed to pollutants. Such other parts include, for example, the neck, scalp, arms, legs and torso.

In a preferred embodiment, the invention comprises the use of a polymer consisting of polyacrylamidopropyltrimonium chloride. This compound has the chemical structure:

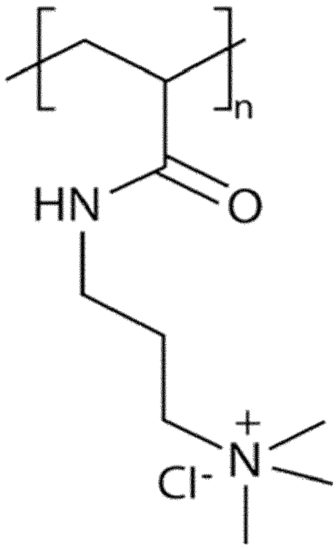

and is commercially available from Ashland, USA under the trade mark N-DurHance A-1000 polymer, and is used as an ingredient in shampoos and conditioners.

The value of n in the formula above is typically in the range 500-2,500, preferably in the range 1000-2,000, and most preferably in the range 1,200-1,800.

In another embodiment, the polymer is a co-polymer of acrylamidopropyltrimonium salt and acrylamide. Such a co-polymer is commercially available from Ashland, USA under the trade mark N-DurHance AA 2000. This polymer material is similar to N-DurHance A-1000 and is approved as an ingredient for compositions for topical application.

In yet another embodiment, the invention may utilise a mixture of two or more different polymers, each polymer comprising an acrylamidopropyltrimonium salt. For example, in one embodiment the invention may utilise a mixture comprising two polymers, especially a mixture comprising a first polymer consisting of polyacrylamidopropyltrimonium salt, and a second polymer being a co-polymer of acrylamide and acrylamidopropyltrimonium salt. As an illustration, the invention may utilise a mixture of Ashland N-DurHance A-1000 and Ashland N-DurHance AA 2000. The two polymers may be combined in any suitable ratio (e.g. from 20:1 to 1:20) and, given the teaching of the present specification, the person skilled in the art could determine a preferred ratio and percentage content of the two polymers in a formulation without exercise of inventive activity.

Typically the polymer comprising an acrylamidopropyltrimonium salt for use in the invention has an average molecular greater than 50 kDa, preferably greater than 100 kDa, more preferably greater than 150 kDa, and most preferably greater than 200 kDa. Conveniently the polymer comprising the acrylamidopropyltrimonium salt of use in the invention has an average molecular weight of less than 1,000 kDa, preferably less than 750 kDa, and most preferably less than 500 kDa. A preferred average molecular weight for the polymer comprising the acrylamidopropyltrimonium salt of use in the invention lies in the range 250-500 kDa, more preferably 250-400 kDa.

The polymer comprising the acrylamidopropyltrimonium salt is conveniently present in a composition formulated for topical application to human skin, scalp or hair. Such compositions may be formulated as a cleaning formulation such as a liquid soap, a gel, a shampoo, a conditioner or the like; or as a cosmetic such as a face cream, face spray, body spray, body lotion, moisturizer, foundation or the like. The formulation of such personal care compositions is well-known to those skilled in the art and forms no part of the present invention.

The amount of polymer comprising acrylamidopropyltrimonium salt in the personal care composition may vary within quite wide limits, such as from 0.5% v/v to 10.0% v/v, but to obtain optimal anti-lipoperoxidation activity it is considered that an amount in the range 1.0-5.0% v/v is likely to be preferable, and an amount in the range 2.0-4.0% v/v is most preferable.

The personal care compositions of use in the present invention will typically include a dermatologically acceptable carrier. Such a dermatologically acceptable carrier is a diluent that does not cause significant irritation to the skin and does not negate or interfere with the anti-pollutant properties of the polyacrylamidopropyltrimonium salt active agent.

Examples of dermatologically acceptable carriers which may be suitable include, but are not limited to, water, such as deionized or distilled water, emulsions, such as oil-in-water or water-in-oil emulsions, alcohols, such as ethanol, isopropanol or the like, acetone, glycols, such as propylene glycol, glycerin or the like, creams, aqueous solutions, oils ointments, pastes, gels, lotions, milks, foams, suspensions, powders, or mixtures thereof. In some embodiments, the composition contains from about 99.95 to about 50% v/v of the dermatologically acceptable carrier.

The personal care composition may also contain other ingredients, such as emollients (e.g. hydrocarbon oils, esters, natural oils, or fatty acids), surfactants, rheology modifiers, humectants, waxes, sensory modifiers, preservatives/antioxidants/chelating agents, reducing agents, pH adjusting agents/buffers/neutralizing agents, sunscreen actives, vitamins, proteins/amino acids, plant extracts, natural ingredients, bio-actives, fragrances/perfumes, deodorizing actives, topical medicament actives, infrared (IR)-absorbing materials, acne medications, volatiles/propellants. The amount of such optional ingredients effective for achieving the desired property provided by such ingredients can be readily determined by one skilled in the art.

In practising the method of the invention, the personal care compositions are generally administered topically by applying the compositions onto the skin in a conventional manner, such as by rubbing or massaging the skin with the composition. A person of ordinary skill in the art can readily determine an appropriate frequency with which the compositions should be applied. The frequency may depend, for example, on the amount of pollution that an individual is encountering in a given day. By way of nonlimiting example, administration of the composition at least once per day may be suitable.

It is known that polyacrylamidopropyltrimonium salts are capable of forming a film. Without being bound by any particular theory, it is hypothesised that the ability to form a thin barrier on human skin may be involved in the mechanism by which the compound acts to protect skin against pollution or other agents that mediate lipoperoxidation.

The invention will now be further described by way of illustrative example and with reference to the accompanying drawings, in which:

Figure 1 is a bar chart showing the results of an MDA assay conducted using human skin explants exposed to a variety of test substances in the absence (left hand half) or presence (right hand half) of mixed pollutants; and

Figure 2 is a bar chart showing the difference, in the amount of MDA detected in the culture medium of human skin explants treated with a variety of test substances, between presence and absence of pollutants.

## Detailed description of the invention

## Examples

Summary - a study was conducted to evaluate the anti-pollution activity of various test substances, by applying the test substances to living human skin explants and then exposing the explants to a pollutant mixture.

The anti-pollutant activity was evaluated by:

i) histological assessment of cellular viability after Masson's trichome staining of the explants; and
ii) performing a biomedical assay of malondialdehyde ("MDA") - a marker of oxidative stress generated by lipid peroxidation.

Materials and Methods

**Table 1**

| Sample Reference | Test Substance |
|---|---|
| S1 | V - 15 |
| S2 | V - 5 |
| S3 | V - 1 |
| S4 | A-1000 - 3 |
| S5 | A-1000 - 1.75 |
| S6 | A-1000 - 0.5 |
| S7 | W |
| S8 | X - 0.5 |
| S9 | X - 0 |
| S10 | Y |
| S11 | Z |

The personnel conducting the tests were not informed of the identity of the test substances (i.e. they were performed "blind"), so that there was no risk of unconscious bias in interpreting the results. It can be seen that samples S1-S3 contained the same test substance, "V", but at three difference concentrations Similarly samples S4-S6 all contained the same test substance ("A-1000") but at three concentrations (3%, 1.75% and 0.5% w/v respectively). Test substance A-1000 was polyacrylamidopropyltrimonium chloride, with an average molecular weight of about 300 kDa.

The various samples were all stored at room temperature during the study.

Skin Explant Collection

99 human skin explants of an average diameter of 12mm (±1mm) were prepared using an abdominal plasty from a 53-year old Caucasian woman. The explants were maintained in BEM culture medium in an incubator at 37°C in a humid, 5%-$CO_2$ atmosphere. BEM is an abbreviation for Bio-EC's Explant Medium. The formulation of BEM is proprietary to Bio-EC. However qualitatively comparable results can be obtained using other media which are commercially available, such as Dulbecco's Modified Eagle Medium ("DMEM").

Sample Application to Explants

On day zero (D0) and day one (D1), samples S1-S11 were topically applied to the explants, 2μl per explant (about 2mg sample per $cm^2$ of explant), and the samples spread over the explants using a small spatula, 4 explant

replicates per sample. Control explants did not receive any treatment. All explants (sample treated and control) underwent replacement of the BEM culture medium (2ml per well) on day 2, following pollutant exposure (described below).

Pollutant Mixture

The pollutant formulation applied to the explants comprised a mixture of heavy metals, hydrocarbons and diesel particulates as follows:

**Heavy Metals**

- Al 0.01mg/ml
- AS 0.01mg/ml
- B 0.001mg/ml
- Ba 0.001mg/ml
- Be 0.0005mg/ml
- Ca 0.005mg/ml
- Cd 0.001mg/ml
- Cr 0.001mg/ml
- Cu 0.0001mg/ml
- Fe 0.001mg/ml
- Hg 0.0025mg/ml
- K 0.495mg/ml
- Li 0.001mg/ml
- Mg 0.0005mg/ml
- Mn 0.0005mg/ml
- Na 0.01mg/ml
- Ni 0.0025mg/ml
- P 0.005mg/ml
- Pb 0.01mg/ml
- Sc 0.0005mg/ml
- Sa 0.01mg/ml
- Sr 0.0005mg/ml
- Te 0.01mg/ml
- Ti 0.001mg/ml
- Y 0.0005mg/ml
- Zn 0.001mg/ml

**Hydrocarbons**

- Benzene 1μl (Fluka, Ref. 12550)
- Xylene 1μl (Fluka, Ref. 95673)
- Toulene 1μl (Sigma, Ref. 34866)

**Diesel particles**

NIST (National Institute of Standards and Technology, USA) ref. SRM1650b

- Diesel particulates 0.01%

Pollutant exposure

On day 2 (D2) and day 3 (D3), explants selected for pollutant exposure were placed in the PolluBox® system (BIO-EC, 1 Chemin de Saulxier, Longjumeau, France) with 900 μl per well of Hanks' Balanced Salt Solution (HBBS), and exposed to 3ml of a vaporized mixture of polycyclic aromatic hydrocarbons, heavy metals and particulate matter

supplemented with NaCl 0.9% (150 µl of NaCl 0.9% per ml of pollutant solution) for 1.5 hours.

The control explants were kept in 1 ml of HBSS during this time. On D2, at the end of the pollutant exposure, all the explants were put back in 2ml of fresh BEM medium. In each case, at the end of pollutant exposure, the test and control explants were put back in the BEM medium renewed on D2.

Sampling

On Day 0, three control explants were collected and cut in two parts. Half was fixed in buffered formalin and the other half was frozen at -80°C. On D4 (24 hours after the second pollutant exposure), three explants from each batch were collected and treated the same way as described above. The fourth explant was kept frozen at -80°C.

The BEM culture medium from all explants (2 ml) was harvested on D4 (24 hours after the second pollutant exposure) and frozen at -20°C for subsequent MDA assay.

Histological processing

After fixation for 24 hours in buffered formalin, the samples were dehydrated and impregnated in paraffin using a Leica PEARL dehydration automat. The samples were embedded using a Leica EG 1160 embedding station. 5µm-thick sections were made using a Leica RM 2125 Minot-type microtome, and the sections were mounted on Superfrost® histological glass slides.

The microscopical observations were performed using a Leica DMLB or Olympus BX43 microscope. Pictures were digitized with a numeric DP72 Olympus camera with CellD storing software.

Cell viability assessment

The cell viability of epidermal and dermal structures was observed on paraffinized sections after Masson's trichrome staining, Goldner variant. The cellular viability was assessed by microscopical observation.

Malondialdehyde Assay

As discussed above, malondialdehyde (MDA) is an end product of lipid peroxidation with serious potential to cause damage as a direct consequence of pollution exposure. An assay was therefore performed to assess the ability of the various test substances to prevent pollution driven damage, utilizing MDA as a marker of that damage.

On day 4, levels of MDA in the samples were assessed with an enhanced method of the TBARs (**T**hioBarbituric **A**cid **R**eactive substances) assay. The MDA was assayed in HBSS medium by addition of TBARs solution (thiobarbituric acid, hydrochloric acid and tricholoroacetic acid) and placed in a water bath (80°C for 14 minutes). Substances such as glucose which are not related with lipoperoxidation are known to react with thiobarbituric acid. Therefore, to enhance the specificity of the assay, the MDA was first extracted by a liquid/liquid extraction with butanol. The MD in the butanol extraction was measured by spectrofluorimetry (excitation: 515 nm, emission: 550 nm) using a Tecan Infinite® M200 Pro microplate reader.

**RESULTS**

Cell Viability

As assessed by histological analysis and Masson's trichrome staining, samples 1-9 and 11 did not have any significant effect on cell viability, compared to untreated control explants. In contrast, treatment of the explants with sample 10 did induce slight alterations in the explants, with two cell layers of the epidermis presenting some oedema and the presence of a few cells with nuclei in early stages of pyknosis.

The additional exposure of the explants to the pollutant mixture did not alter the cell viability at all from that described above.

These results show that all of the test samples were well-tolerated by human skin and did not cause any detectable alterations to cell viability, with the sole exception of test sample 10, which caused some slight alterations.

MDA Assay Results

The MDA assay results, based on the amount of MDA detected in the explant culture medium at day 4, are presented in Table 2.

**Table 2**

| **Sample and treatment** | **Mean MDA concentration (nmol/l ± SD)** |
|---|---|
| Negative Control (no test substance; no pollutant) | 140.6 ± 30.6 |
| S1 no pollutant | 178.7 ± 21.2 |
| S2 no pollutant | 156.6 ± 31.3 |
| S3 no pollutant | 125.1 ± 11.0 |
| S4 no pollutant | 132.2 ± 12.5 |
| S5 no pollutant | 145.1 ± 36.9 |
| S6 no pollutant | 108.2 ± 42.3 |
| S7 no pollutant | 147.2 ± 18.7 |
| S8 no pollutant | 154.4 ± 18.1 |
| S9 no pollutant | 131.4 ± 10.4 |
| S10 no pollutant | 195.8 ± 29.5 |
| S11 no pollutant | 111.6 ± 30.3 |
| Positive Control (no test substance; + pollutant) | 536.9 ± 138.3 |
| S1 + pollutant | 312.5 ± 49.9 |
| S2 + pollutant | 323.5 ± 87.3 |
| S3 + pollutant | 269.3 ± 101.8 |
| S4 + pollutant | 124.5 ± 27.0 |
| S5 + pollutant | 347.1 ± 112.8 |
| S6 + pollutant | 273.8 ± 105.1 |
| S7 + pollutant | 305.4 ± 95.1 |
| S8 + pollutant | 369.5 ± 42.4 |
| S9 + pollutant | 263.5 ± 109.6 |
| S10 + pollutant | 265.6 ± 37.6 |
| S11 + pollutant | 157.6 ± 32.8 |

The same results are presented as a bar chart in Figure 1.

Referring to the top portion of Table 2, comparing the MDA assay results of the various test samples 1-11 against the no test control, nearly all of the samples caused a slight increase or decrease in the MDA level, which increase or decrease was <u>not</u> statistically significant as judged by a Student's T-test. The one exception was sample 10, which caused an increase in MDA level of 39% relative to the control, which was significant at the level of $p < 0.1$ in the Student's T-test. It was perhaps noteworthy that sample 10 was also the one sample which appeared to affect cell viability in the histological analysis.

Comparing the MDA level of the negative control (no pollutant) with the positive control (+ pollutant) shows that exposure of the explants to the pollutant mixture induced a highly significant ($p<0.01$) increase in the concentration of MDA in the culture medium.

Comparing the MDA assay results from explants treated with test samples S1-S11 plus pollutant with the positive control explants (no test substance, + pollutant), gives the following results:

Sample S1 induces a significant decrease of 42%*.
Sample S2 induces a significant decrease of 40%*.
Sample S3 induces a significant decrease of 50%*.
Sample S4 induces a significant decrease of 77%**.
Sample S5 induces a significant decrease of 35%$^{\#}$.

Sample S6 induces a significant decrease of 49%*.
Sample S7 induces a significant decrease of 43%*.
Sample S8 induces a significant decrease of 31%#.
Sample S9 induces a significant decrease of 51%*.
Sample S10 induces a significant decrease of 51%*.
Sample 11 induces a significant decrease of 71%**.

Statistical significance: #=p<0.1 (90%) *=p<0.05 (95%) **=p<0.01 (99%)

It is noted that all the test samples caused a significant decrease in MDA concentration relative to the positive control. However, it should be borne in mind that, as shown in the top portion of Table 2, the application of the test samples could itself cause an alteration in the MDA concentration even in the absence of pollutant. Accordingly, the inventors found the mean change in MDA concentration (the "Delta" value, Δ) for each sample according to the simple formula:

$$\Delta = (\text{mean MDA, + pollutant}) - (\text{mean MDA, no pollutant})$$

The calculated Δ values are shown in Table 3.

**Table 3**

| **Sample** | **Δ MDA (nmol/L) ± SD** |
|---|---|
| Control (no test substance) | 396.3 ± 138.3 |
| S1 | 133.7 ± 49.9 |
| S2 | 166.9 ± 87.3 |
| S3 | 144.3 ± 101.8 |
| S4 | -7.8 ± 27.0 |
| S5 | 201.9 ± 112.8 |
| S6 | 165.5 ± 105.1 |
| S7 | 158.2 ± 95.1 |
| S8 | 215.1 ± 42.4 |
| S9 | 132.1 ± 109.6 |
| S10 | 69.8 ± 37.6 |
| S11 | 46.0 ± 32.8 |

The same data are illustrated as a bar chart in Figure 2.

It is immediately apparent from Table 3 that sample S4 gave startlingly good performance in inhibiting MDA production.

Conclusion

The results of the study are summarized in Table 4 below.

**Table 4**

| *Sample* | **Cell viability** | | **MDA Assay** | | |
|---|---|---|---|---|---|
| | *vs -ve control* | *vs +ve control* | *vs -ve control* | *vs +ve control* | *vs Δ control + pollutant* |
| S1 | No change | No change | +27%$^{ns}$ | -42%* | -66%* |
| S2 | No change | No change | +11%$^{ns}$ | -%40* | -58%* |
| S3 | No change | No change | -11%$^{ns}$ | -%50* | -64%* |

(continued)

| *Sample* | **Cell viability** | | **MDA Assay** | | |
|---|---|---|---|---|---|
| | *vs -ve control* | *vs +ve control* | *vs -ve control* | *vs +ve control* | *vs Δ control + pollutant* |
| S4 | No change | No change | -6%$^{ns}$ | -%77** | -102%** |
| S5 | No change | No change | +3%$^{ns}$ | -%35# | -49%# |
| S6 | No change | No change | -23%$^{ns}$ | -%49* | -58%* |
| S7 | No change | No change | +5%$^{ns}$ | -%43* | -60%* |
| S8 | No change | No change | +10%$^{ns}$ | -%31# | -46%# |
| S9 | No change | No change | -7% | -%51* | -67%* |
| S10 | Slight epidermal alterations | Slight epidermal alterations | +39% | -%51* | -82%* |
| S11 | No change | No change | -21%$^{ns}$ | -%71** | -88%* |
| -ve control = control explants with no test substance<br>+ve control = control explants with pollutant exposure<br>ns = not statistically significant<br># = significant with p<0.1 (90%)<br>* = significant with p<0.05 (95%)<br>** = significant with p<0.01 (99%) | | | | | |

It can be seen that sample S4 gave a highly significant reduction in Δ MDA and is thus the best substance, of those tested, in protecting against pollutant exposure-induced lipoperoxidation.

## Claims

1. A polymer comprising an acrylamidopropyltrimonium salt for use in the prevention of pollutant-mediated damage to human skin.

2. The use according to claim 1, wherein the salt is a halide.

3. The use according to claim 1 or 2, wherein the salt is polyacrylamidopropyltrimonium chloride.

4. The use according to any one of the preceding claims, wherein the polymer is, or essentially consists of, polyacrylamidopropyltrimonium salt.

5. The use according to any one of claims 1-3, wherein the polymer is a co-polymer of acrylamidopropyltrimonium salt and acrylamide.

6. The use according to any one of the preceding claims, wherein the polymer comprising an acrylamidopropyltrimonium salt inhibits lipid peroxidation.

7. The use accordingly to any one of the preceding claims, wherein the polymer comprising an acrylamidopropyltrimonium salt is present in a personal care composition formulated for topical application to human skin, scalp or hair.

8. The use according to any one of the preceding claims, wherein the polymer comprising an acrylamidopropyltrimonium salt forms a film on the skin and/or hair of a human individual.

9. The use according any one of the preceding claims, wherein the polymer comprising an acrylamidopropyltrimonium salt is present in an amount of 0.1-5.0% w/v, preferably 0.5-5.0% w/v and more preferably 2.0-5.0% w/v, of a personal care composition formulated for topical application to human skin, scalp or hair.

10. The use according to any one of the preceding claims, wherein the polymer comprising an acrylamidopropyltrimonium salt has an average molecular weight in the range 250-500 kDa, preferably 250-400 kDa.

11. A method for preventing pollutant-mediated damage to human skin, the method comprising the step of applying to the skin of a human subject an effective amount of a polymer comprising an acrylamidopropyltrimonium salt.

12. A method for cosmetic treatment of human skin according to claim 11.

13. The method according to claim 11 or 12, comprising a use in accordance with any one of claims 2-10.

FIGURE 1

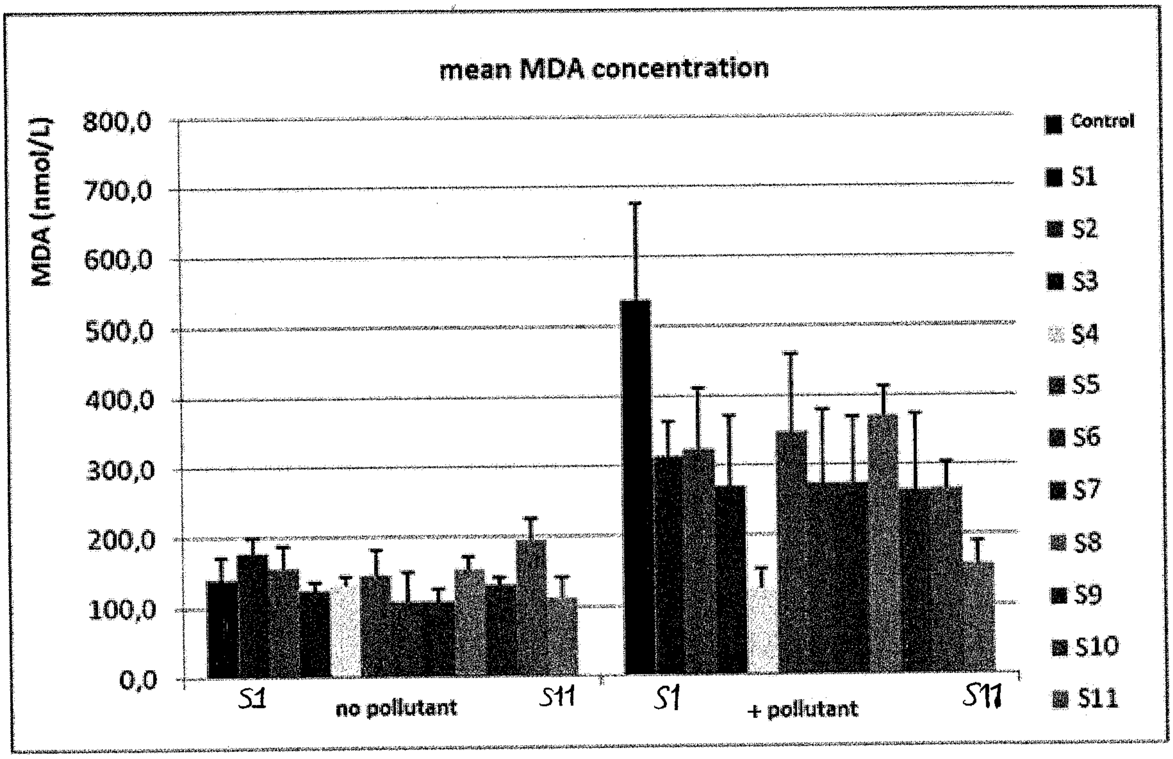
mean MDA concentration
MDA (nmol/L)
800,0
700,0
600,0
500,0
400,0
300,0
200,0
100,0
0,0
S1
no pollutant
S11
S1
+ pollutant
S11
Control
S1
S2
S3
S4
S5
S6
S7
S8
S9
S10
S11

FIGURE 2

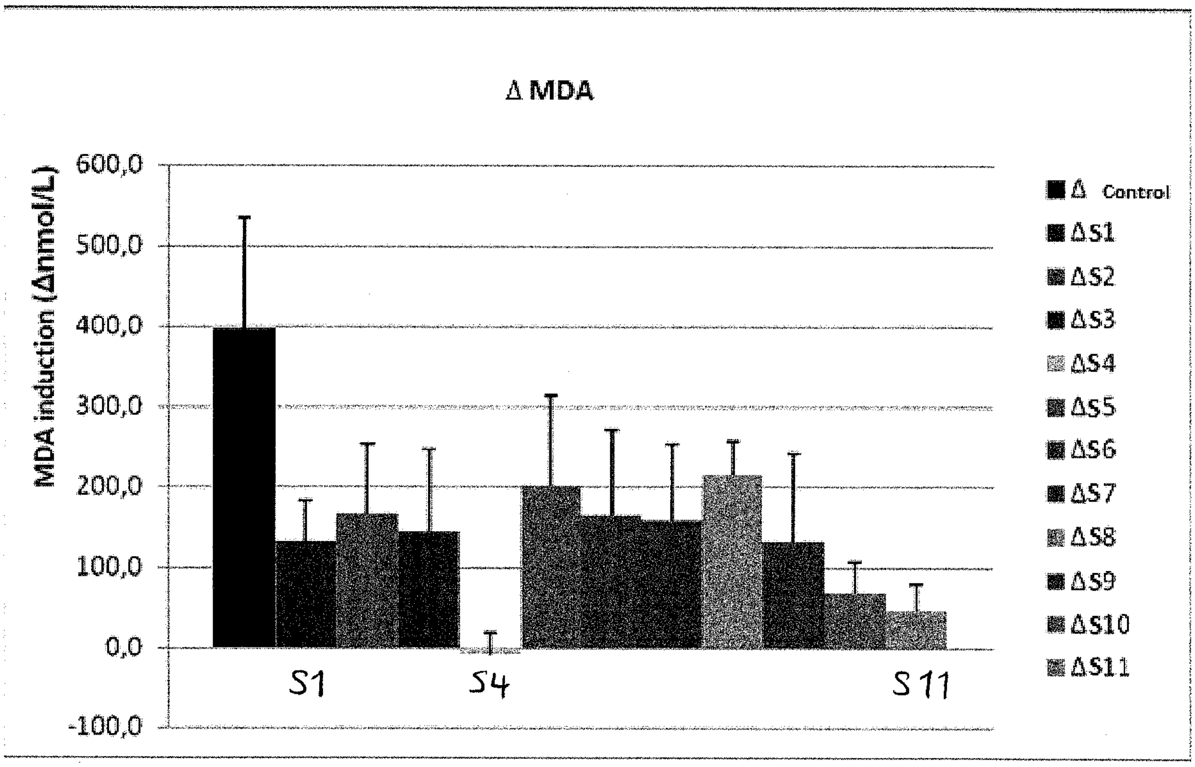
Δ MDA
MDA induction (Δnmol/L)
600,0
500,0
400,0
300,0
200,0
100,0
0,0
-100,0
S1
S4
S11
Δ Control
ΔS1
ΔS2
ΔS3
ΔS4
ΔS5
ΔS6
ΔS7
ΔS8
ΔS9
ΔS10
ΔS11

Europäisches Patentamt
European Patent Office
Office européen des brevets

# EUROPEAN SEARCH REPORT

Application Number
EP 19 21 9354

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIN QU ET AL: "RINSE-OFF PRODUCTS DEFEND HAIR FROM POLLUTION", HOUSEHOLD AND PERSONAL CARE TODAY, TEKNO SCIENZE, MILANO, IT , vol. 13, no. 4 1 July 2018 (2018-07-01), pages 32-36, XP009515542, ISSN: 2035-4614 Retrieved from the Internet: URL:https://www.teknoscienze.com/wp-content/uploads/2018/09/Foltis_HPC4_2018.pdf | 1-9, 11-13 | INV. A61K8/81 A61Q19/00 A61K31/785 A61P17/00 A61P17/18 |
| Y | * abstract * * page 33, column 1 - page 35; figures; table 2 * | 1-13 | |
| Y | WO 97/26860 A1 (JOHNSON & JOHNSON CONSUMER [US]) 31 July 1997 (1997-07-31) * page 1, line 26 - page 2, line 10 * * page 14, lines 3-12 * * examples 4,5,7,8,11,12,14,15,24,26,28,29,38 * * examples 40,42,43,45,46,48,50-52 * | 1-13 | |
| Y | EP 3 097 905 A1 (SYMRISE AG [DE]) 30 November 2016 (2016-11-30) * the whole document * | 1-13 | |
| Y | WO 2006/058755 A1 (UNILEVER PLC [GB]; UNILEVER NV [NL] ET AL.) 8 June 2006 (2006-06-08) * page 3, line 7 - page 4, line 2 * * page 12, line 29 - page 13, line 12 * | 1-13 | |
| | -/-- | | |

TECHNICAL FIELDS SEARCHED (IPC)
A61K
A61Q
A61P

The present search report has been drawn up for all claims

2

| Place of search | Date of completion of the search | Examiner |
|---|---|---|
| Munich | 5 June 2020 | Ruckebusch, Virginie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 21 9354

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2013/174615 A2 (UNILEVER NV [NL]; UNILEVER PLC [GB]; CONOPCO INC DBA UNILEVER [US]) 28 November 2013 (2013-11-28) * the whole document * ----- | 1-13 | |
| A | US 9 693 948 B1 (POHLAD DONNA [US] ET AL) 4 July 2017 (2017-07-04) * the whole document * ----- | 1-13 | |

TECHNICAL FIELDS SEARCHED (IPC)

The present search report has been drawn up for all claims

2

| Place of search | Date of completion of the search | Examiner |
|---|---|---|
| Munich | 5 June 2020 | Ruckebusch, Virginie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 21 9354

This annex lists the patent family members relating to the patent documents cited in the above-mentioned European search report. The members are as contained in the European Patent Office EDP file on The European Patent Office is in no way liable for these particulars which are merely given for the purpose of information.

05-06-2020

| Patent document cited in search report | Publication date | Patent family member(s) | Publication date |
|---|---|---|---|
| WO 9726860 A1 | 31-07-1997 | AU 718594 B2 | 20-04-2000 |
| | | CA 2245010 A1 | 31-07-1997 |
| | | CN 1214628 A | 21-04-1999 |
| | | DE 69714016 D1 | 22-08-2002 |
| | | DE 69714016 T2 | 13-03-2003 |
| | | EP 0879047 A1 | 25-11-1998 |
| | | WO 9726860 A1 | 31-07-1997 |
| EP 3097905 A1 | 30-11-2016 | BR 112017025221 A2 | 07-08-2018 |
| | | BR 112017025275 A2 | 07-08-2018 |
| | | BR 112017025331 A2 | 31-07-2018 |
| | | BR 112017025424 A2 | 07-08-2018 |
| | | CN 107847411 A | 27-03-2018 |
| | | CN 107847412 A | 27-03-2018 |
| | | CN 107847413 A | 27-03-2018 |
| | | CN 107912022 A | 13-04-2018 |
| | | EP 3097905 A1 | 30-11-2016 |
| | | JP 2018516259 A | 21-06-2018 |
| | | JP 2018516260 A | 21-06-2018 |
| | | JP 2018516261 A | 21-06-2018 |
| | | JP 2018520119 A | 26-07-2018 |
| | | KR 20180005260 A | 15-01-2018 |
| | | KR 20180005261 A | 15-01-2018 |
| | | KR 20180006454 A | 17-01-2018 |
| | | KR 20180006455 A | 17-01-2018 |
| | | US 2018147135 A1 | 31-05-2018 |
| | | US 2018153783 A1 | 07-06-2018 |
| | | US 2018256473 A1 | 13-09-2018 |
| | | US 2018360733 A1 | 20-12-2018 |
| | | WO 2016188988 A1 | 01-12-2016 |
| | | WO 2016189024 A1 | 01-12-2016 |
| | | WO 2016189038 A1 | 01-12-2016 |
| | | WO 2016189046 A1 | 01-12-2016 |
| WO 2006058755 A1 | 08-06-2006 | AR 052040 A1 | 28-02-2007 |
| | | EP 1817080 A1 | 15-08-2007 |
| | | JP 2008521860 A | 26-06-2008 |
| | | US 2008096786 A1 | 24-04-2008 |
| | | WO 2006058755 A1 | 08-06-2006 |
| WO 2013174615 A2 | 28-11-2013 | BR 112014027633 A2 | 27-06-2017 |
| | | CN 109010128 A | 18-12-2018 |
| | | EP 2872109 A2 | 20-05-2015 |
| | | JP 6518187 B2 | 22-05-2019 |
| | | JP 2015517541 A | 22-06-2015 |
| | | JP 2018127483 A | 16-08-2018 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 19 21 9354

This annex lists the patent family members relating to the patent documents cited in the above-mentioned European search report. The members are as contained in the European Patent Office EDP file on The European Patent Office is in no way liable for these particulars which are merely given for the purpose of information.

05-06-2020

| Patent document cited in search report | Publication date | Patent family member(s) | Publication date |
|---|---|---|---|
| | | US 2015110728 A1 | 23-04-2015 |
| | | WO 2013174615 A2 | 28-11-2013 |
| US 9693948 B1 | 04-07-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2017100311 A


### Non-patent literature cited in the description


- *Cosmetics,* 2017, vol. 4, 57
- **JANERO ; FREE RADIC.** *Biol. Med.,* 1990, vol. 9 (6), 515-540
- **PORTUGAL-COHEN et al.** *Clinical, Cosmetic and Investigational Dermatology,* 2017, vol. 10, 185-193